# EUROPEAN PATENT SPECIFICATION

(11) **EP 0 487 617 B1**
(45) Date of publication and mention of the grant of the patent: **02.10.1996**
(21) Application number: 90913186.4
(22) Date of filing: 13.08.1990
(51) Int. Cl.: A61K 38/00, C07K 4/00, C07K 16/00

(54) **SYNTHETIC PEPTIDES THAT ENHANCE CELL BINDING**
KÜNSTLICHE PEPTIDE, DIE DIE ZELLBINDUNG STEIGERN
PEPTIDES SYNTHETIQUES POUVANT AMELIORER LA LIAISON DE CELLULES

(30) Priority: 14.08.1989 US 393621
(43) Date of publication of application: 03.06.1992
(73) Proprietor: THE REGENTS OF THE UNIVERSITY OF CALIFORNIA, Oakland, California 94612-3550 (US)
(72) Inventor: BHATNAGAR, Rajendra, Sahai, Burlingame, CA 94010 (US)
(74) Representative: Kirschner, Klaus Dieter, Dipl.-Phys.
(86) International application number: US9004538
(87) International publication number: WO9102537

(56) References cited:
- EDITOR: RIVIER ET AL // AUTHOR: SCARIA ET AL 'PEPTIDES - Proceedings of the 11th American Peptide Symposium, July 9-14, 1989; La Jolla, California' 1990 , ESCOM , LEIDEN, HOLLAND
- CHEMICAL ABSTRACTS, vol. 87, 1977, Columbus, Ohio, US; abstract no. 1690, MASUI ET AL: 'Synthetic substrates for vertebrate collagenase' page 1679 ;column 2 ;
- Gene, Volume 39 (2-3), issued 1985, FRENCH et al., "Nucleotide sequence of a cDNA clone of a mouse pro 1 (I) collagen protein", pages 311-312, see page 312.
- Biochemistry, Volume 22, issued 22 April 19838 BERNARD et al., "Nycleotide Sequences of Complementary Deoxyribonucleic Acids for the Pro 1 Chain of Human Type I Procollagen. Statistical Evaluation of Structures Thar Are Conserved during Evolution", pages 5213-5223, see page 5217 (Fig. 3).

## Description

This invention relates to a method of promoting vertebrate cell adhesion comprising attaching a composition comprising an amino acid sequence of GTPGPQGIAGQRGVV, or a portion thereof having an enhanced cell binding activity, or an antiidiotypic antibody reactive with an antibody for at least one epitope defined by the said amino acid sequence or a portion thereof, to a substrate and adding cells to the substrate. Additionally, the present invention relates to a substrate to be used with such method and to antiidiotypic antibodies used therewith.

This invention was made with Government support under Grant No. AR-37267 awarded by the Department of Health and Human Services. The Government has certain rights in this invention.

### Brief Description of the Relevant Art

Collagen is the most abundant protein found in vertebrates. Approximately twenty-five percent of all animal protein is collagen. Collagen is unusual among proteins in that the amino acid glycine constitutes one-third of the total amino acid content and occurs at nearly every third amino acid residue. Also, many proline amino acids are found in collagen. Collagen contains two amino acids present in very few other proteins, i.e., hydroxyproline and hydroxylysine. The sequence glycine-proline-hydroxyproline recurs frequently.

Because glycine is a very small amino acid, chains of collagen can wind tightly around one another to form a triple helix. The side chains of proline form cross-links that lock the three strands together. Additionally, mature collagen frequently contains carbohydrate units covalently attached to its hydroxylysine residues. A disaccharide of glucose and galactose is commonly found attached to strands of collagen. Still other forms of collagen can form planar sheets, which are rich in carbohydrates. For a general description of the structure of collagen, see Stryer, Biochemistry (3d ed.), W.H. Freeman & Co., San Francisco (1988); Ramachandran, et al. (eds.), Biochemistry of Collagen, Plenum Press, New York (1976); Mayne, et al. (eds), Structure and Function of Collagen Types, Academic Press, New York (1987).

Collagen functions as a structural protein of tissues. It is the major fibrous element in skin, cartilage, bone, tendon, teeth, and blood vessels. Collagen is present to some extent in nearly all organs and serves to hold cells together in discrete units. It forms insoluble fibers that have a high tensile strength. Furthermore, the basic structure of collagen is modified to meet the specialized needs of particular tissues, and these are reflected in the various types of collagen that have been identified.

The several types of collagen are a family of genetically related proteins that exhibit fundamentally similar secondary and tertiary protein structures, see Mayne, et al. (eds.), Structure and Function of Collagen Types, Academic Press, New York (1987). As used herein unless otherwise specified, "collagen" refers to any of the known types of collagens.

Type I collagen, the most prevalent type and the species found in skin, tendon, bone, and cornea, is comprised of two chains of one kind, termed α1(I), and one of another, termed α2(I). Other types of collagen have three identical chains. Each of the three strands consists of about 1,000 amino acid residues, and each has a helical conformation. The three strands wind around each other to form a superhelical cable and are hydrogen bonded to each other. As mentioned above, this structure is possible because of the presence and regularity of glycine units.

Studies have shown that the presence and concentration of imino residues, proline and hydroxyproline, are essential for generating and stabilizing the triple helical conformation of collagen, see Bhatnagar, et al., pp. 479-53 in Ramachandran, et al. (eds.), Biochemistry of Collagen, Plenum Press, New York (1976); Bhatnagar, et al., pp. 429-38 in Agris (ed.), Biomolecular Structure and Function, Academic Press, New York (1978).

In short, the stability of the helical form of a single strand of collagen depends on the locking effect of proline and hydroxy-proline residues. The triple helix is further stabilized by transverse hydrogen bonding and van der Waals interactions between residues on different strands. The superhelix is sterically allowed because glycine occupies every third position in the amino acid sequence.

In addition to being a major determinant of the architecture and tensile strength of tissues, collagen participates in numerous physiologically important interactions. These include, but are not limited to, the formation of complexes with other macromolecules such as fibronectin, the modulation of cell proliferation, the mediation of cell migration and differentiation, and the modulation of specific gene expression.

In order for such interactions to occur, the molecules on the surface of collagen fibers must exhibit molecular perspectives that are specific for recognition. This requires local conformational changes. It has been suggested that the binding of certain cells, such as platelets, may involve a conformationally perturbed region of the α1 chain of collagen, which is located approximately one-quarter of the length of the chain from the C-terminus, see Dessau, et al., Biochem. J. 169:55-59 (1978); Kleinman, et al., J. Biol. Chem. 253:5642-46 (1978). This region also includes the only site known to be susceptible to proteolytic cleavage by the vertebrate enzyme, collagenase, see Gross, pp. 275-317 in Ramachandran, et al., (eds.), Biochemistry of Collagen, Plenum Press, New York (1976); Miller, et. al., Biochem. 15:787-92 (1976). Additionally, this region is known to be involved in the binding of fibronectin to collagen, see Dessau, et al., Biochem. J. 169:55-59 (1978); Kleinman, et al., Biochem. & Biophys. Res. Comm. 72:426-32 (1976); Kleinman, et al., Analyt. Biochem. 94:308-12 (1979); and is also the site of intermolecular interactions leading to fibril formation, see Silver, J. Biol. Chem. 256:4973-77 (1981).

Previous studies have shown that the 3 amino acid sequence, RGD, found in a variety of proteins, including collagen, may play a major role in the binding of cells, see Dedhar, et al., J. Cell Biol. 104:585-93 (1987); Pierschbacher, et al., J. Cell. Biochem. 28:115-26 (1985). This sequence appears twice within the α1(I) chain, and one of those occurrences is within the conformationally perturbed region described above, see Kleinman, et al., J. Biol. Chem. 253:5642-46 (1978).

/From Peptides - Proceedings of the 11 th American Peptides Symposium, July 9-14, 1989; La Jolla, CA, 1990, ESCOM, Leiden, Holland, as well as Chemical Abstracts 87 (1977), no. 1690 and Tetrahedron Letters 29, 4341-4344 (1988) peptides having the sequence GTPGPQGIAGQRGVV are known. From BBA 445, 521 (1976), Biochemistry 24, 6730 (1985), J. Biol. Chem. 262, 6222 (1987) short peptides comprising the sequences QGIAGQ or GIAG are known. DNP-peptides of PLGIAGR-NH₂ and PQGIAGQ-D-Arg are commercially available.

### SUMMARY OF THE INVENTION

The present invention makes use of synthetic compositions of matter that are structurally or biologically analogous to a small region of collagen, including all or part of 15 amino acid residues, GTPGPQGIAGQRGVV, of the α1(I) chain of collagen, referred to herein as P-15. The compositions include peptides, wherein the peptides will usually have a primary sequence that comprises at least 4 contiguous amino acids within P-15. The compositions used in the present invention are especially useful for promoting vertebrate cell adhesion to a substrate by coating it with the compositions.

### DETAILED DESCRIPTION OF THE INVENTION AND PREFERRED EMBODIMENTS

Novel synthetic compositions of matter are described in the present invention. These include compounds that are structurally or biologically analogous to a small region of collagen. This region, referred to herein as P-15, includes all or part of 15 amino acid residues, GTPGPQGIAGQRGVV, of the α1(I) chain of collagen and spans approximately residues 766-780 of this chain. (The one-symbol amino acid abbreviations are used herein, see Stryer, Biochemistry (3d ed.), W.H. Freemen & Co., San Francisco (1988).) P-15 does not occur as a natural fragment of collagen nor is it a product of natural enzymatic cleavages.

As mentioned above, collagen exists in a very tightly coiled superhelical structure, wherein its tensile strength is stabilized by the high content of imino residues. Thus, for interactions with other cells or compounds, the collagen structure must be perturbed. Thermal motion can overcome the forces that stabilize the triple-stranded helix, yielding a disrupted structure. A major advantage of the present invention is that, given the size and structure of the synthetic compositions disclosed herein, there is no need for conformational perturbations, and these compounds not only can mimic the biological activity of collagen but also can, in at least some cases, exhibit enhanced cell binding functions.

The synthetic compounds of this invention include peptides. Peptides described herein include sequences comprised of at least P-15, and sequences comprised of at least 4 contiguous amino acids within P-15. Also included are antiidiotypic antibodies to antibodies to P-15 or a portion thereof. These peptide compositions, as well as their methods of preparation, are described below.

Synthetic peptide compounds used in the present invention will usually include all or part of a 15 amino acid residue, termed P-15 and comprising GTPGPQGIAGQRGVV.

The amino acid sequences of these synthetic peptides need not correspond precisely to the P-15 sequence but rather may include only a portion of it. At least 4 contiguous amino acids, i.e., QGIA, GIAG, or IAGQ, must be present and preferably will include at least the entire QGIAGQ sequence. The peptides will usually contain 100 or fewer amino acids, more usually 50 or fewer amino acids, and typically 25 or fewer amino acids. A preferred peptide has up to 15 amino acids and the most preferred sequence is P-15. Additionally, more amino acids can be present at either the N-terminus or C-terminus.

The peptides of the present invention may also embody substitutions of particular amino acids, although there often will be no substitutions in peptides containing only 3 or 4 amino acids. Normally, there will be no more than one substitution in sequences from 5 to about 10 amino acids, and no more than two substitutions in sequences having a length from about 10 to 20 amino acids. Substitutions, of course, must not substantially alter the activity of the peptide in an adverse manner.

A further embodiment of this invention involves synthetic peptides consisting in part or in all of nonnatural amino acids. Such nonnatural amino acids usually will have at least an N-terminus and a C-terminus and will have side chains that are either identical to, or chemically modified or substituted from, their natural amino acid counterparts. An example of a nonnatural amino acid is an optical isomer of a naturally occurring L-amino acid. Examples of chemical modifications or substitutions include hydroxylation or fluorination of C-H bonds within natural amino acids. Such techniques are used to manufacture drug analogs of biological compounds and are known to one of ordinary skill in the art.

The synthetic peptides described above are preferably substantially free of the following: natural folding, glycosylation, association with other peptide chains, cross-linking, and hydroxylation. These properties tend to be present in naturally occurring collagen. By "substantially free," it is meant less than the average amount found in naturally occurring collagen.

Synthetic peptides that have biological activity the same or similar to that of natural sequences may be produced by either of two general approaches.

First, polypeptides having fewer than about 100 amino acids, usually fewer than about 50 amino acids, and more usually fewer than about 25, may be synthesized by the well-known Merrifield solid-phase chemical synthesis method wherein amino acids are sequentially added to a growing chain, see Merrifield, J. Am. Chem. Soc. 85:2149-56 (1963). Although there is no theoretical upper limit to the size of the peptides of the present invention, there will seldom be a reason to prepare peptides having a length greater than 100 amino acids, and the peptides often will be shorter than 50 amino acids. Linear peptides may be chemically synthesized by manual means or by automation in commercially available synthesis equipment. Systems for manually synthesizing peptides on polyethylene pegs are available from Cambridge Research Biochemicals, Cambridge, Massachusetts. Automatic peptide synthesis equipment is available from suppliers, including Applied Biosystems, Inc., Foster City, California; Beckman Instruments, Inc., Waldwick, New Jersey; and Biosearch, Inc., San Rafael, California. Using such automatic synthesizers according to manufacturer's instructions, peptides may be produced in gram quantities for use in the present invention.

The use of relatively short, linear peptide fragments is advantageous in performing the methods of use described in the present invention. The peptides may be produced in quantity and will be free from contaminating substances, which are often found in peptides produced by recombinant techniques.

Second, the synthetic peptides of the present invention may be synthesized by recombinant techniques involving the expression in cultured cells of recombinant DNA molecules encoding the gene for a desired portion of the α1(I) strand of collagen. The gene encoding the desired portion of the α1(I) strand of collagen itself may be natural or synthetic. Conveniently, polynucleotides may be synthesized by well-known techniques. For example, short single-stranded DNA fragments may be prepared by the phosphoramidite method described by Beaucage, et al., Tetrahedron Letters 22:1859-62 (1981). A double-stranded fragment then may be obtained either by synthesizing the complementary strand and annealing the strands together under appropriate conditions or by adding the complementary strand using DNA polymerase with an appropriate primer sequence. The natural or synthetic DNA fragments coding for the desired portion of the α1(I) strand of collagen will be incorporated in DNA constructs capable of introduction to and expression in an in vitro cell culture.

Usually, the DNA constructs will be suitable for replication in a unicellular host, such as yeast or bacteria, but also may be intended for introduction and integration within the genome of cultured mammalian or other eukaryotic cell lines. DNA constructs prepared for introduction into bacteria or yeast will include a replication system recognized by the host, the DNA fragment encoding for the desired α1(I) polypeptide, transcriptional and translational initiation regulatory sequences joined to the 5'-end of the α1(I) collagen DNA sequence, and transcriptional and translational termination regulatory sequences joined to the 3'-end of the α1(I) collagen sequence. The transcriptional regulatory sequences will include a heterologous promoter that is recognized by the host. Conveniently, available expression vectors that include the replication system and transcriptional and translational regulatory sequences, together with an insertion site for the α1(I) collagen DNA sequence, may be employed.

For use in cell binding, these peptides are obtained in a substantially pure form, that is, typically about 50% w/w or more purity, substantially free of interfering proteins and contaminants. Preferably, the peptides are isolated or synthesized in a purity of at least about 80% w/w and, more preferably, in at least about 95% w/w purity. Using conventional purification techniques, homogeneous peptides of at least 99% w/w can be obtained. For example, the peptides may be purified by use of reverse-phase high performance liquid chromatography. Usually, however, it is not essential to obtain highly pure, i.e., 99%, peptides when using these peptides to bind cells.

Biological activities that may be possessed by the peptide include inhibition of collagen synthesis, inhibition of collagen binding, and inhibition of cell migration. Of particular interest to the present invention is the property of enhanced cell binding.

In another embodiment, monoclonal antibodies may be raised against an epitopic region defined by P-15 or a portion thereof, or by any of the compounds of the present invention, wherein the epitopic region is responsible for binding and biological activity. By then raising antibodies against the first antibody, the binding region of the antiidiotypic antibodies may provide functional analogs of P-15 or a functionally similar compound described above.

Such antibodies may be obtained by injecting the purified peptide or nonpeptide compound into a wide variety of vertebrates in accordance with conventional techniques. Suitable vertebrates include mice, rats, sheep, and goats; in particular, mice. Often, the animals are bled periodically with successive bleeds having improve titer and specificity. The compound may be injected intramuscularly, intraperitoneally, subcutaneously, or the like. Often, a vehicle is employed, such as a complete or incomplete Freund's adjuvant. If desired, monoclonal antibodies can be prepared. Alternatively, the compound may be used to isolate monospecific antibodies from a natural source.

To obtain monoclonal antibodies, spleen cells from the immunized vertebrate are immortalized. The manner of immortalization is not critical. Presently, the most common method is fusion with a myeloma fusion partner. Other techniques include EBV transformation; transformation with bare DNA, e.g., oncogenes, retroviruses, etc.; or any other method that provides for stable maintenance of the cell line and production of monoclonal antibodies. Human monoclonal antibodies may be obtained by fusion of the spleen cells with an appropriate human fusion partner. A detailed technique for producing mouse x mouse monoclonal antibodies is taught by Oi, et al., pp. 351-72 in Mishell, et al. (eds.), Selected Methods in Cellular Immunology, W.H. Freeman & Co., San Francisco (1980). The antibodies of the present invention may be from any immunoglobulin class.

The present invention also describes methods of use for promoting vertebrate cell adhesion comprising attaching any of the above-described compositions of matter to a substrate and adding cells to the substrate. Substrates include, but are not limited to, glass, plastics, ceramics, organic polymers, gels, and silica. Preferred types of cells to be adhered include fibroblasts; however, most, if not all, cell types may be used.

The mode of attachment can be via covalent linkages, noncovalent interactions, or nonspecific adsorption. Covalent linkages include, but are not limited to, those involving ester, amide, or ether, see Carey, et al., Advanced Organic Chemistry, Part B, Plenum Press, New York (1983). An exemplary method of covalent linkages involves peptides of the present invention with additions of nonnatural amino acids at either the N-terminus or C-terminus to provide for binding or conjugation of the peptide to a solid phase or another protein. For example, a cysteine sequence may be added to either terminus to facilitate coupling to a carrier. Hydrophobic residues or lipid-containing moieties, such as amino acids containing hydrophobic side chains, may be added to enhance liposome or membrane binding. The substrate of choice may be pretreated with CnBr or other activating reagents to facilitate coupling of the composition to the substrate. Noncovalent interactions and nonspecific adsorption typically would involve the direct application of a solution containing the present compositions to the substrate. These methods of use have many applications, e.g., culturing cells to study their physiology and to make specific compounds, etc. Such advantages are readily apparent to one of ordinary skill in the art.

### EXAMPLES

The following examples are intended to he merely illustrative of the present invention and are not to be read as limiting. Example 1 demonstrates the enhanced promotion of cell adhesion by P-15 as compared with collagen. Example 2 illustrates comparative inhibition of cell binding to collagen in the presence of P-15 and other peptides.

### EXAMPLE 1

The peptide P-15, GTPGPQGIAGQRGVV, of the present invention was synthesized by solid phase procedures using symmetrical anhydrides for coupling of Boc-amino acids, except for Q residues that were coupled with l-hydroxybenzyl triazole. Circular dichroism and ¹H and ¹³C NMR studies suggest that P-15 exists in a random conformation in aqueous solutions and has little tendency to generate collagen or polyproline-like conformations in solution in the presence of helicogenic solvents but shows a tendency to form a β-strand structure in decreasingly polar solvents.

All media and supplies for tissue culture were obtained from University of California San Francisco Tissue Culture Facility. Cycloheximide was purchased from Sigma Biochemicals, St. Louis, Missouri. Rat tail tendon collagen was purified to homogeneity and characterized in our laboratory using published methods. Tissue culture dishes (35 mm diameter, Falcon Plastics) were coated with collagen dissolved in 0.01 N acetic acid to a concentration of 10 µg/ml. The dishes were allowed to air dry overnight under ultraviolet light to maintain sterility.

Primary cultures of fibroblast cells from 19-week fetal human dermis were obtained. The cultures were maintained in Eagles minimum essential medium with Earle's salts supplemented with 10% fetal bovine serum. All cells used were in the second passage and were obtained from confluent cultures. Prior to release and dispersion with trypsin, the cells were rinsed three times with phosphate buffered saline. The cell layers were exposed for 2 minutes to 0.05% trypsin, 0.02% NaEDTA in phosphate buffered saline, and rinsed with serum free medium.

Glass coverslips (12-15) were washed in a cleaning solution overnight then rinsed with large amounts of distilled water. The coverslips were then sterilized by drying between sheets of filter paper in an oven at high temperature. The coverslips were then placed in small culture dishes under sterile conditions. A solution of collagen in acetic acid (1 mg/ml) was prepared. Fifty microliters of solution was placed on each of four coverslips, which were then dried in air overnight. A solution of P-15 in acetic acid (1mg/ml) was prepared. Four coverslips were treated with 50µl of the solution and then dried as described above.

Approximately 100,000 fibroblast cells were pipetted onto 4 control coverslips and dishes, 4 collagen coated coverslips and dishes, and 4 P-15 coated coverslips and dishes. After 15 minutes, excess medium containing floating cells was removed from each dish. Fresh medium was added and the dishes were examined under a microscope to determine the extent of binding of cells to the respective coverslips. If the number of bound cells appeared to be different, an assay for DNA in the cells attached to each coverslip was performed. The cells were counted using a hemocytometer.

| Relative Binding of Cells After 15 Minutes | |
|---|---|
| | Concentration of Cells |
| Control (no collagen or P-15) | 7.01 |
| Collagen | 8.59 |
| P-15 | 11.05 |

These results demonstrate an increase of 60% in cell binding of P-15 over the control (no collagen or P-15) dishes and an increase of 40% in cell binding of P-15 over collagen-coated dishes.

### EXAMPLE 2

Collagen related peptides (PPG)₁₀ and (P-Hyp-G)₁₀ (wherein Hyp is hydroxyproline) were obtained from Peninsula Laboratory, Belmont, CA. Polyproline was purchased from Sigma Biochemicals, St. Louis, Missouri. Dynorphin 1-13 (YGGFLRRIRPKLK) and human preangiotensinogen 1-14 (DRVYIHPFHLVIHN) were purchased from Bachem, Torrance, CA. Fibronectin related peptide sequences (RGD, GRDSP, and GRGDTP) were also obtained from Bachem and were used without further analysis.

P-15 was synthesized as described in Example 1 and the same procedures and conditions of Example 1 were used herein.

In this experiment to examine the effect of various additives on binding, the additives were added in the medium containing the cells before being transferred to the dishes for the binding assay. It was observed that cell binding to collagen proceeds rapidly in the beginning of the experiment but then slows down after approximately 30 minutes and approximately 70-80% of the cell binding in the absence of a peptide occurs during this time. The effect of the peptide inhibitor on the initial rate of the binding reaction between cells and collagen was examined. The effects of various conditions on cell binding to collagen after 30 minutes were usually compounded.

The inhibition of cell binding by P-15 was specific for the peptide, because a number of other peptides failed to show the same effect, see results presented below. In order to ascertain if inhibition of binding by peptides may be a nonspecific process, the effect of equimolar concentrations (35 x 10⁻⁶M) of several peptides on the binding of cells to collagen was compared. All peptides used had small inhibitory effects on cell binding, although none reached the level of inhibition observed with P-15. As seen in the results, polyproline, (PPG)₁₀ and (P-Hyp-G)₁₀₋, peptides with collagen-related conformations, did not significantly affect the binding of cells. Under the conditions of these experiments, polyproline and (PPG)₁₀ exist as single chains in polyproline conformation whereas (P-Hyp-G)₁₀ is a triple helix. The behavior of dynorphin 1-13 and preangiotensinogen 1-14, peptides similar in size to P-15, on cell binding was also examined. They did not affect the binding of cells to collagen to the extent shown by P-15. These results show that P-15 is a potent inhibitor of the binding of cells to collagen.

| Effect of P-15 and Other Peptides on the Attachment of Cells to Collagen Substrates | | |
|---|---|---|
| | Percent of Maximal Cell Attachment | Percent Inhibition of Cell Attachment |
| Control (No addition) | 100 | -- |
| P-15, 7.2 x 10⁻⁶M | 15 | 85 |
| P-15, 36 x 10⁻⁶M | 12 | 88 |
| (PPG)₁₀^{a}, 35 x 10⁻⁶M | 79 | 21 |
| (P-Hyp-G)₁₀^{b}, 35 x 10⁻⁶M | 87 | 13 |
| Polyproline^{c}, 1.7 x 10⁻⁶M | 90 | 10 |
| Dynorphin 1 - 13, 35 x 10⁻⁶M | 81 | 19 |
| Preangiotensinogen 1-14, 35 x 10⁻⁶M | 88 | 12 |

| | | |
|---|---|---|
| ^{a}Under the conditions of this experiment, (PPG)₁₀ is not a triple helix. (Tₘ in H₂0 is 24.5°C.) | | |
| ^{b}Under the conditions of this experiment, (P-Hyp-G)₁₀ is a triple helix. | | |
| ^{c}MW approx. 2,700 kilodaltons. | | |

## Claims

1. A method of promoting vertebrate cell adhesion comprising attaching a composition comprising an amino acid sequence of GTPGPQGIAGQRGVV, or a portion thereof having an enhanced cell binding activity, or an antiidiotypic antibody reactive with an antibody for at least one epitope defined by the said amino acid sequence or a portion thereof, to a substrate and adding cells to the substrate.

2. The method of claim 1, wherein the amino acid sequence is selected from QGIA, GIAG and IAGQ.

3. The method of claim 1, wherein the amino acid sequence is QGIAGQ.

4. The method of claim 1, wherein the amino acid sequence is GTPGPQGIAGQRGVV (P-15).

5. The method of claim 1, wherein the composition comprises an antiidiotypic antibody.

6. The method of claim 5, wherein the cells are fibroblasts.

7. An antiidiotypic antibody reactive with an antibody for an epitope defined by the amino acid sequence of GTPGPQGIAGQRGVV, or a portion thereof as defined in any of claims 1 to 3 having an enhanced cell binding activity to vertebrate cells.

8. A substrate comprising a coating composition promoting vertebrate cell adhesion, said coating composition comprising an amino acid sequence of GTPGPQGIAGQRGVV, or a portion thereof having an enhanced cell binding activity, or an antiidiotypic antibody reactive with an antibody for an epitope defined by said amino acid sequence or portion thereof.

## Patentansprüche

1. Verfahren zur Förderung der Adhäsion von Wirbeltierzellen durch Anheften einer Zusammensetzung, die eine Aminosäurensequenz GTPGPQGIAGQRGVV oder einen Teil davon mit erhöhter Zellbindungsaktivität oder einen antiidiotypischen Antikörper, der mit wenigstens einem Antikörper für wenigstens ein durch diese Aminosäurensequenz oder einen Teil davon definiertes Epitop reaktiv ist, umfaßt, an ein Substrat und Zugeben von Zellen zu dem Substrat.

2. Verfahren nach Anspruch 1, worin die Aminosäurensequenz aus QGIA, GIAG und IAGQ ausgewählt ist.

3. Verfahren nach Anspruch 1, worin die Aminosäurensequenz QGIAGQ ist.

4. Verfahren nach Anspruch 1, worin die Aminosäurensequenz GTPGPQGIAGQRGVV (P-15) ist.

5. Verfahren nach Anspruch 1, worin die Zusammensetzung einen antiidiotypischen Antikörper umfaßt.

6. Verfahren nach Anspruch 5, worin die Zellen Fibroblasten sind.

7. Antiidiotypischer Antikörper, der mit einem Antikörper für ein durch die Aminosäurensequenz GTPGPQGIAGQRGVV oder ein Teil davon, wie in einem der Ansprüche 1 bis 3 definiert, mit erhöhter Zellbindungsaktivität an Wirbeltierzellen, definiertes Epitop reaktiv ist.

8. Substrat, das eine die Adhäsion von Wirbeltierzellen fördernde Beschichtungszusammensetzung aufweist, wobei diese Beschichtungszusammensetzung eine Aminosäurensequenz GTPGPQGIAGQRGVV oder ein Teil davon mit erhöhter Zellbindungsaktivität oder einen antiidiotypischen Antikörper, der mit einem Antikörper für ein durch diese Aminosäurensequenz oder ein Teil davon definiertes Epitop reaktiv ist, umfaßt.

## Revendications

1. Procédé pour favoriser l'adhésion cellulaire de vertébrés, comprenant la fixation d'une composition, comprenant une séquence aminoacide de GTPGPQGIAGQRGVV, ou une portion de celle-ci ayant une activité de liaison cellulaire augmentée, ou un anticorps antiidiotypique réactif avec un anticorps pour au moins un épitope défini par ladite séquence aminoacide ou par une portion de celle-ci, à un substrat et en ajoutant des cellules à ce substrat.

2. Procédé selon la revendication 1, dans lequel la séquence aminoacide est choisie parmi QGIA, GIAG et IAGQ.

3. Procédé selon la revendication 1, dans lequel la séquence aminoacide est QGIAGQ.

4. Procédé selon la revendication 1, dans lequel la séquence aminoacide est GTPGPQGIAGQRGVV (P-15).

5. Procédé selon la revendication 1, dans lequel la composition comprend un anticorps antiidiotypique.

6. Procédé selon la revendication 5, dans lequel les cellules sont des fibroblastes.

7. Un anticorps antiidiotypique réactif avec un anticorps pour un épitope défini par la séquence aminoacide de GTPGPQGIAGQRGVV, ou une portion de celle-ci, comme décrit dans l'une quelconque des revendications 1 à 3 ayant une activité de liaison cellulaire augmentée pour des cellules de vertébrés.

8. Un substrat comprenant une composition de revêtement pour favoriser l'adhésion cellulaire de vertébrés, ladite composition de revêtement comprenant une séquence aminoacide de GTPGPQGIAGQRGVV, ou une portion de celle-ci ayant une activité de liaison cellulaire augmentée, ou un anticorps antiidiotypique réactif avec un anticorps pour un épitope défini par ladite séquence aminoacide ou une portion de celle-ci.
